# FASCICULE DE BREVET EUROPEEN

(11) **EP 1 039 855 B1**
(45) Date de publication et mention de la délivrance du brevet: **23.06.2004**
(21) Numéro de dépôt: 98962522.3
(22) Date de dépôt: 18.12.1998
(51) Int. Cl.: A61F 2/44, A61F 2/46

(54) **PROTHESE DISCALE PARTIELLE**
PARTIELLE BANDSCHEIBENPROTHESE
PARTIAL DISCAL PROSTHESIS

(30) Priorité: 19.12.1997 FR 9716548; 12.03.1998 FR 9803268
(43) Date de publication de la demande: 04.10.2000
(73) Titulaire: Sofamor Danek Holdings, Inc., Memphis, TN 38132 (US)
(72) Inventeur: Graf, Henry, 69006 Lyon (FR)
(74) Mandataire: Neyret, Daniel
(86) Numéro de dépôt international: PCT/FR1998/002798
(87) Numéro de publication internationale: WO 1999/032054

(56) Documents cités:
- EP-A- 0 260 044
- EP-A- 0 346 269
- EP-A- 0 566 810
- EP-A- 0 610 837
- WO-A-90/11740
- WO-A-95/00082
- WO-A-95/15133
- WO-A-97/15247
- WO-A-97/31517
- DE-C- 4 323 595
- US-A- 5 616 142

## Description

La présente invention concerne une prothèse discale partielle.

De manière habituelle, les prothèses discales, qui peuvent être partielles ou totales, sont destinées à remplacer tout ou partie d'un disque intervertébral lorsque ce dernier a été détruit par la chirurgie ou par la maladie.

Un premier type de prothèse discale consiste en une cage rigide, qui peut être par exemple de section transversale rectangulaire, dans laquelle sont ménagées des perforations propres à recevoir des greffons destinés à assurer une solidarisation satisfaisante de cette cage avec les deux vertèbres entre lesquelles elle doit être insérée. Ce type de cage rigide, qui est implantée notamment par impactage ou par vissage, présente un inconvénient en ce sens qu'elle conduit à un blocage complet des deux vertèbres entre lesquelles est disposée la cage, ce qui limite la liberté de mouvement du patient.

On connaît également, par le document EP-A-0 346 269, une prothèse de disque intervertébral formée par une âme en matériau viscoélastique intercalée entre deux plaques de recouvrement métalliques, qui sont destinées à être en contact, une fois implantées, avec la surface des vertèbres. Il existe cependant un inconvénient lié à ce type-de prothèse, qui réside notamment dans son manque de stabilité, de sorte que cette prothèse possède de forts risques d'être éjectée de l'espace intervertébral.

Le document EP-A-0610837 décrit une prothèse discale présentant toutes les caractéristiques du préambule de la revendication 1.

Afin de pallier les inconvénients de l'art antérieur évoqués ci-dessus, l'invention se propose de réaliser une prothèse discale partielle, qui puisse être antérieure ou postérieure, dont la mise en place dans l'espace intervertébral soit aisée, qui possède une stabilité satisfaisante au sein de cet espace intervertébral et qui permette une liberté de mouvement suffisante tout en garantissant le maintien d'une posture physiologiquement avantageuse.

A cet effet, l'invention a pour objet une prothèse discale partielle destinée à être insérée entre deux vertèbres voisines, comprenant une âme réalisée en un matériau élastique tel qu'un polymère de silicone ou un élastomère, recouverte, sur une partie de sa périphérie, par un enrobage réalisé en un matériau rigide et destiné à être en contact avec lesdites deux vertèbres voisines, caractérisée en ce que ladite âme comprend, en coupe transversale, deux portions d'extrémité reliées par une portion médiane, ledit enrobage comprend deux coiffes pourvues d'un filetage et recouvrant au moins partiellement la périphérie externe desdites portions d'extrémité, et la distance séparant lesdites coiffes augmente vers la partie antérieure de la prothèse.

L'invention se propose également de réaliser un outil de pose pour la prothèse telle que décrite ci-dessus, qui assure une implantation aisée de cette dernière, qui puisse être facilement retiré une fois la prothèse implantée et qui permette de préserver l'intégrité des différents organes au voisinage desquels cet outil est déplacé durant l'ensemble de ces opérations.

A cet effet, l'invention a également pour objet un outil de pose pour la prothèse telle que décrite ci-dessus, caractérisé en ce qu'il comprend un manche de préhension prolongé par des moyens propres à solidariser ladite prothèse par rapport audit outil dans un état transversalement comprimé de ladite prothèse.

L'invention va être décrite ci-dessous, en référence aux dessins annexés, donnés uniquement à titre d'exemple non limitatif et dans lesquels :
- la figure 1 est une vue en perspective partiellement arrachée d'un premier mode de réalisation d'une prothèse discale conforme à l'invention ;
- les figures 2 et 3 sont des vues éclatées en bout représentant respectivement les parties avant et arrière de la prothèse représentée à la figure 1 ;
- la figure 4 est une vue schématique en perspective, avec arrachement, d'un outil de pose pour la prothèse représentée aux figures 1 à 3 ;
- la figure 5 est une vue en perspective montrant la prothèse des figures 1 à 3 engagée dans l'outil de pose de la figure 4 ;
- la figure 6 est une vue en coupe suivant la ligne VI-VI à la figure 5 ;
- la figure 7 est une vue schématique illustrant l'implantation de la prothèse des figures 1 à 3 ;
- la figure 8 est une vue schématique illustrant la désolidarisation de l'outil de pose de la figure 4, par rapport à la prothèse des figures 1 à 3, après implantation de cette dernière ;
- la figure 9 est une vue schématique en perspective d'une prothèse conforme à un deuxième mode de réalisation de l'invention ;
- la figure 10 est une vue en coupe axiale d'une prothèse conforme à un troisième mode de réalisation de l'invention ;
- la figure 11 est une vue en coupe axiale d'un quatrième mode de réalisation d'une prothèse discale conforme à l'invention ;
- les figures 12 et 13 sont des vues en bout représentant respectivement les parties avant et arrière de la prothèse de la figure 1 ;
- la figure 14 est une vue schématique en perspective, avec arrachement, des différents éléments constitutifs d'un outil de pose pour la prothèse représentée aux figures 11 à 13 ;
- la figure 15 est une vue en coupe longitudinale illustrant la solidarisation de l'outil de pose de la figure 4 et de la prothèse des figures 11 à 13 ;
- la figure 16 est une vue partielle en bout, illustrant les languettes dont est pourvue la prothèse représentée aux figures 11 à 13, lorsque cette dernière est implantée ;
- la figure 17 est une vue analogue à la figure 16, illustrant la position respective des languettes de la prothèse en vue du retrait de cette dernière ;
- la figure 18 est une vue en perspective d'un cinquième mode de réalisation d'une prothèse discale conforme à l'invention ;
- les figures 19 et 20 sont des coupes longitudinales suivant la ligne XIX-XIX à la figure 18, dans des positions respectivement de repos et de compression de la prothèse de cette figure 18 ;
- les figures 21 et 22 sont des coupes transversales suivant la ligne XXI-XXI à la figure 18, dans des positions respectivement de repos et de compression de la prothèse de cette figure 18 ;
- la figure 23 est une vue en perspective avec arrachement d'une âme composite d'une prothèse conforme à l'invention et
- la figure 24 est une vue en coupe longitudinale d'une âme formée de plusieurs éléments appartenant à une prothèse conforme à l'invention.

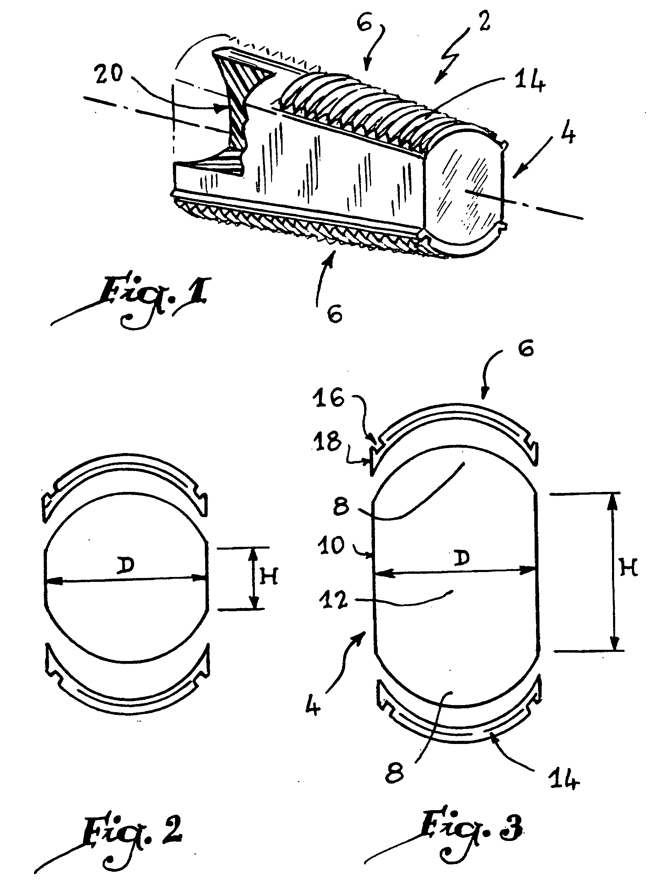

Comme le montrent les figures 1 à 3, la prothèse discale conforme à l'invention et désignée dans son ensemble par la référence 2, comprend une âme 4 dont la surface extérieure est partiellement recouverte au moyen de deux enrobages formées par des coiffes 6. L'âme 4 est réalisée en un matériau élastique biocompatible, comme par exemple un polymère de silicone ou un caoutchouc précontraint. Les coiffes 6 sont exécutées en un matériau rigide biocompatible tel que par exemple en acier spécial, notamment du titane, et sont assujetties à l'âme par exemple par une colle de silicone ou autre.

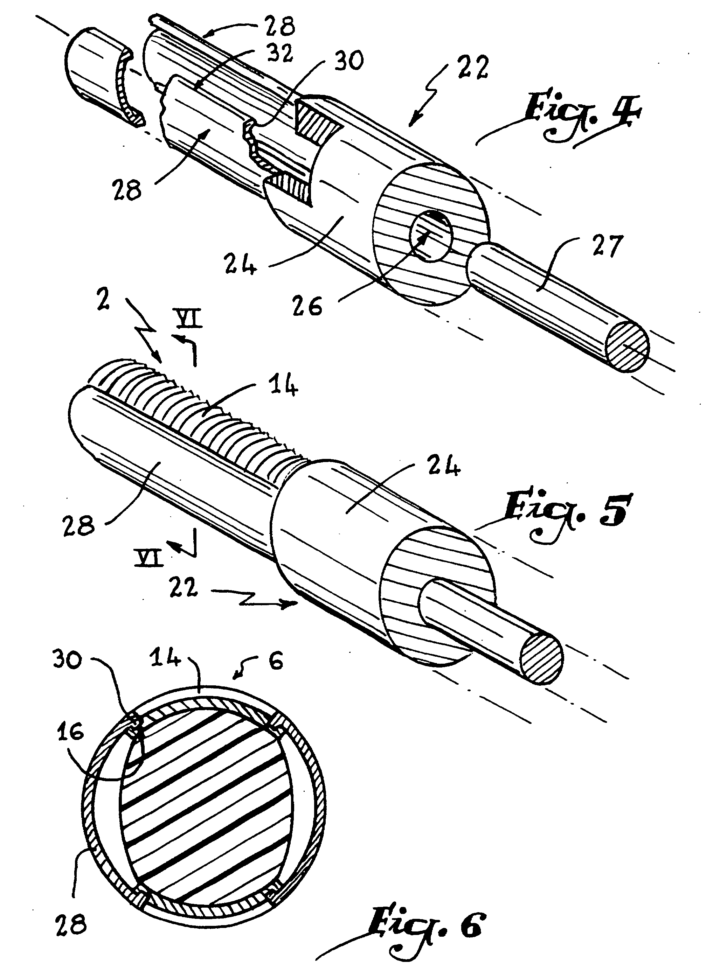

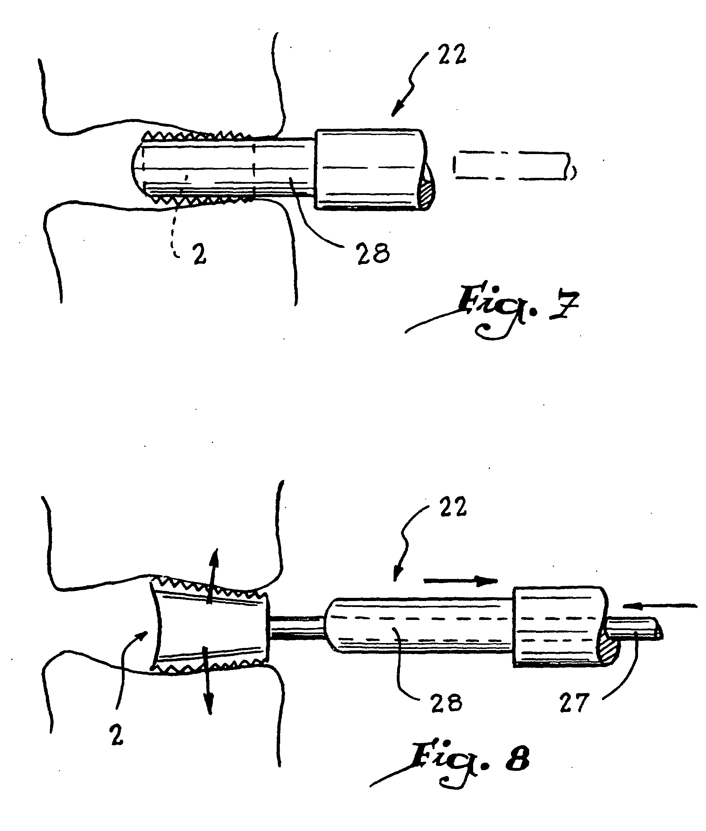

Comme le montrent en particulier les figures 2 et 3, la section de l'âme 4 se compose de deux portions d'extrémités 8 dont la périphérie extérieure décrit un arc de cercle, qui sont reliées par deux méplats 10 formant une partie médiane 12.

Un renfoncement 20 en forme de coupelle est ménagé dans l'extrémité avant de l'âme 4, et constitue une amorce de flexion, comme cela sera visible dans la suite de la description.

Chaque coiffe 6 est réalisée sous forme d'un profilé présentant, en coupe transversale, une forme d'arc de cercle. Ces coiffes recouvrent la totalité de la périphérie extérieure des portions d'extrémités 8 de l'âme 4, alors que les méplats 10 ne sont pas recouverts. La surface extérieure de ces coiffes est munie d'un filetage 14 destiné à faciliter l'implantation de la prothèse, comme cela sera explicité dans ce qui suit.

La surface extérieure des coiffes 6 possède en outre des irrégularités formées par exemple par gaufrage ou frittage, qui sont destinées à garantir une bonne stabilité de la prothèse une fois montée. Des rainures axiales 16 sont en outre ménagées sur la totalité de la longueur de chaque coiffe, au voisinage de chaque bordure 18 de ces dernières.

Il convient de noter que la dimension transversale ou largeur D des portions d'extrémité 8 est sensiblement constante tout le long de la prothèse, alors que la dimension ou hauteur H des méplats 10 reliant ces portions d'extrémité, augmente vers l'avant de la prothèse, en faisant référence à la prothèse une fois montée sur un patient.

La distance séparant les coiffes augmente vers l'avant de la prothèse. On entend par distance séparant les coiffes la distance maximale, en coupe transversale, séparant, dans la position comprimée de la prothèse, les zones de contact respectives des coiffes avec les vertèbres.

A titre indicatif, la longueur de la prothèse, à savoir la distance séparant ses extrémités avant et arrière, est par exemple de l'ordre de 16 à 20 mm, sa hauteur H minimale au niveau de la partie arrière de la prothèse, est de l'ordre de 12 mm, alors que sa hauteur H maximale est de l'ordre de 16 mm. Le rayon de courbure de la partie intérieure de chaque coiffe est par exemple voisin de 12 mm, et ces coiffes s'étendent sur un secteur angulaire de l'ordre de 120° chacune. Enfin, l'épaisseur des coiffes est par exemple de 2 mm environ.

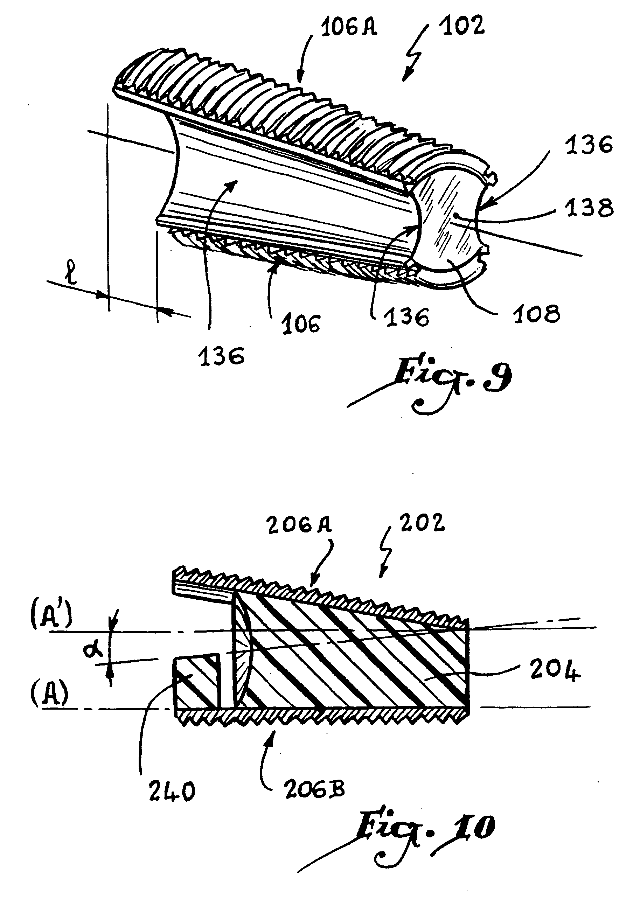

La figure 4 représente un outil désigné dans son ensemble par la référence 22, destiné à la pose de la prothèse 2 au sein de l'espace intervertébral d'un patient. Cet outil 22 comprend un manche cylindrique 24 allongé dont les dimensions sont appropriées pour conférer une préhension aisée par un chirurgien. Ce manche 24 est percé d'un orifice central co-axial 26, dont les dimensions transversales sont inférieures à celles de la partie arrière de la prothèse 2. Cet orifice 26 est propre au passage d'une tige 27, comme cela sera explicité dans ce qui suit.

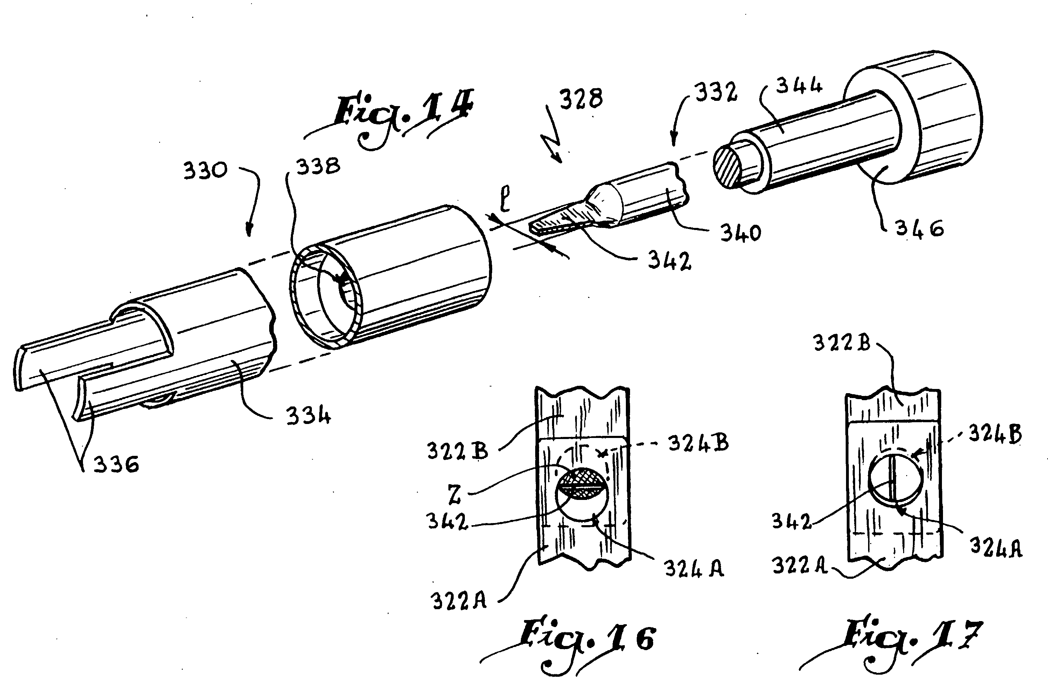

Le manche 24 est terminé par deux languettes 28 symétriques l'une de l'autre réalisées sous forme d'un profilé mince en forme d'arc de cercle. Ces languettes possèdent un rayon de courbure voisin de celui des coiffes 6 de la prothèse et s'étendent sur des secteurs angulaires dont la valeur, ajoutée à celle des secteurs angulaires des coiffes 6, est légèrement supérieure à 360°. Des nervures axiales 30 font saillie vers l'intérieur sur l'intégralité de la longueur de chaque languette, au niveau de chacune des bordures 32 de celle-ci, comme le montre en particulier la figure 6. Les dimensions transversales de ces nervures sont telles qu'elles sont propres à se loger dans les rainures 16 ménagées dans les coiffes 6.

La dimension longitudinale des languettes 28 est analogue, voire très légèrement supérieure, à celle de l'ensemble de la prothèse 2.

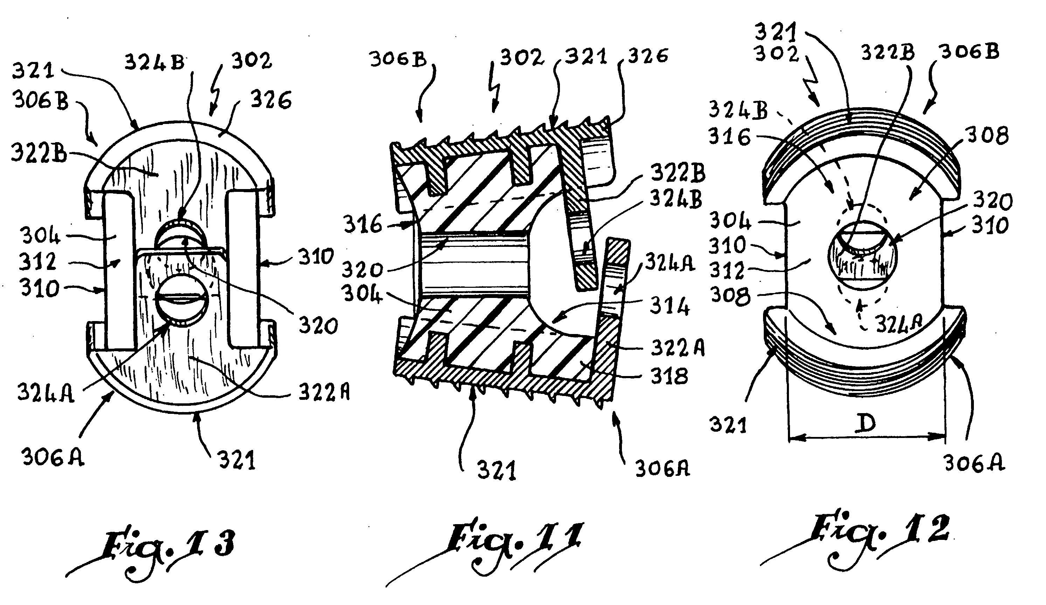

Les figures 5 et 6 représentent la prothèse 2 engagée dans le volume cylindrique défini par les languettes 28 de l'outil de pose 22. En vue du montage de la prothèse, il convient tout d'abord d'engager l'extrémité des nervures 30, opposée au manche 24, dans l'extrémité arrière des rainures 16. Puis, il s'agit de comprimer, par exemple manuellement, l'avant de la prothèse, ce qui est possible à la fois du fait de la nature élastique de l'âme 4 et de la présence du renfoncement 20. Puis, on fait coulisser chaque nervure 30 au sein d'une rainure 16 correspondante, jusqu'à ce que l'extrémité arrière de la prothèse 2 vienne en butée contre l'extrémité du manche 24 adjacente aux languettes 28.

Une fois montée de cette manière, la prothèse maintenue sous forme cylindrique a subi une diminution globale de ses dimensions transversales du fait de la compression, de plus en plus sensible en allant vers l'avant de la prothèse. Comme le montre en particulier la figure 6, la partie médiane 12 de la prothèse est en particulier soumise à des efforts tendant à diriger sa périphérie extérieure vers les languettes 28 de l'outil de pose.

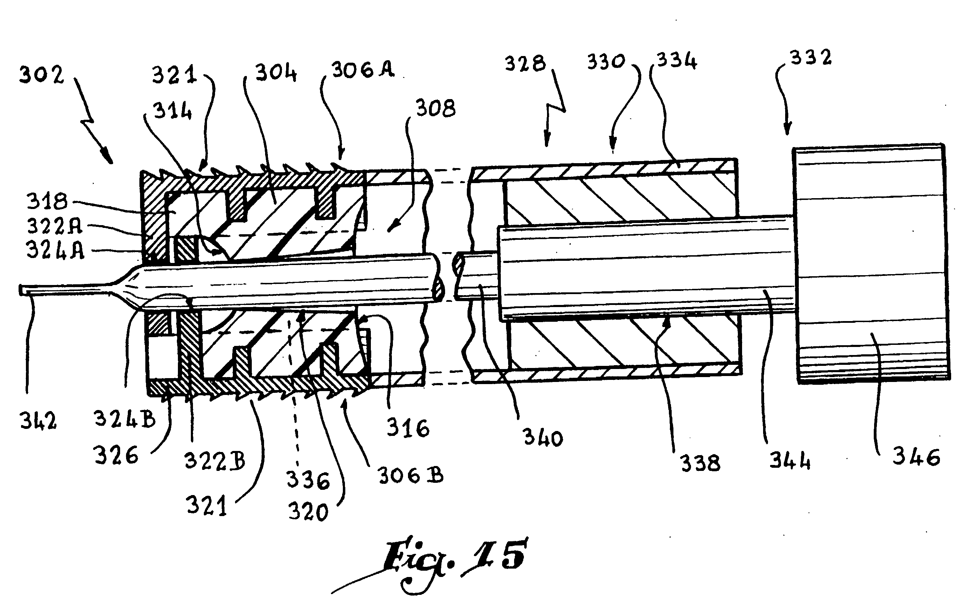

L'ensemble composé de la prothèse comprimée et des languettes 28 de l'outil de pose présente une forme globalement cylindrique. Comme le montre la figure 7, le montage de la prothèse 2 s'effectue par vissage au moyen du manche 4 de l'outil de pose 22. Le filetage 14 dont est pourvue la surface extérieure des coiffes 6 est avantageux à l'égard de cette opération.

La surface extérieure des languettes 28 est avantageusement lisse car l'outil de pose doit être retiré après montage.

La figure 8 illustre l'opération consistant à retirer l'outil de pose 22 de la prothèse 2. A cet effet, une fois que cette dernière est implantée en un emplacement approprié, on la maintient axialement au moyen de la tige 27 pénétrant dans l'orifice 26 ménagé dans le manche 24. Puis, on fait coulisser vers l'arrière les nervures 30 des languettes 28 le long des rainures 16 des coiffes 6. La prothèse recouvre alors sa forme originelle, telle que représentée aux figures 1 à 3, du fait de la nature de son matériau constitutif précontraint.

Les figures 5 à 8 illustrent l'implantation d'une prothèse 2 destinée à être placée à l'arrière de l'espace intervertébral et donc à constituer une prothèse partielle postérieure. On peut également prévoir que cette prothèse 2 soit positionnée à l'avant de cet espace intervertébral. A cet effet, il convient de solidariser, par rapport à l'outil de pose 22, cette prothèse en disposant sa partie antérieure évasée de manière adjacente au manche 24. La pose de cette prothèse est ensuite effectuée par vissage, comme dans l'exemple décrit en référence à la figure 7. Cette implantation est réalisée depuis la partie antérieure du patient, par exemple par coelioscopie.

La figure 9 représente une prothèse 102 conforme à un second mode de réalisation de l'invention. L'âme 104 de cette dernière comprend, de manière analogue à celle 4 décrite en référence aux figures précédentes, deux portions d'extrémité 108 dont la périphérie extérieure décrit un arc de cercle. Ces portions 108 sont reliées, non plus par des méplats, mais au moyen de gorges 136, de sorte que les portions d'extrémités 108 constituent des portions renflées reliées au moyen d'une partie médiane en forme de col 138. Chaque portion d'extrémité 108 est recouverte au moyen de coiffes 106 formant enrobage, dont l'une 106A s'étend longitudinalement au-delà de l'extrémité avant de l'âme 104, sur une longueur l. Cette coiffe 106A est destinée à constituer la coiffe supérieure, une fois la prothèse implantée. La coiffe supérieure 106A fait saillie par rapport à la partie avant de l'âme 104 d'environ quelques millimètres.

Les autres éléments constitutifs de cette prothèse 102 auxquels il n'est pas fait allusion dans la présente description, sont identiques à ceux de la prothèse 2 décrite précédemment.

La figure 10 représente une prothèse 202 conforme à un troisième mode de réalisation de l'invention.

Cette prothèse 202 comporte deux enrobages formés par des coiffes 206A et 206B respectivement supérieure et inférieure de dimensions axiales équivalentes.

La coiffe supérieure 206A fait saillie au-delà de l'extrémité avant de l'âme 204, de manière analogue à la prothèse 102.

Une butée 240 fait saillie à partir de la coiffe inférieure 206B en direction de la coiffe supérieure 206A. Les dimensions de cette butée sont telles qu'elle limite le mouvement d'inclinaison de la coiffe supérieure 206A à une valeur déterminée par rapport à un axe (A') parallèle à l'axe (A) de la coiffe inférieure. Par exemple, l'inclinaison maximale de la coiffe supérieure 206A peut être limitée à environ 5° (angle α) vers le bas, par rapport à l'axe (A').

La butée 240 est réalisée en un matériau analogue à celui constituant l'âme 204, et peut être venue de matière avec cette dernière. Les éléments non décrits de cette prothèse sont analogues à ceux de la prothèse 102.

On peut prévoir qu'un renfoncement analogue à celui 20 soit ménagé dans la partie arrière de la prothèse conforme à l'invention. Toutefois, ce renfoncement doit alors présenter des dimensions sensiblement inférieures au renfoncement 20, de sorte qu'une flexion préférentielle est réalisée au niveau de la partie avant de la prothèse.

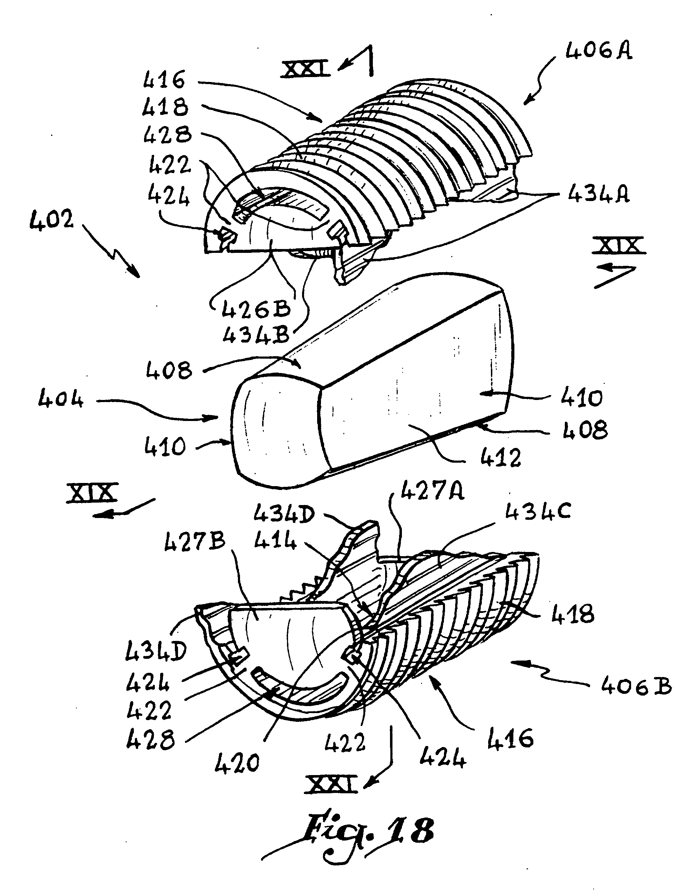

Les figures 11 à 13 représentent un quatrième mode de réalisation d'une prothèse discale partielle conforme à l'invention, désignée dans son ensemble par la référence 302. Cette prothèse comprend une âme 304 réalisée en un matériau élastique biocompatible, comme par exemple un polymère de silicone ou un caoutchouc pré-contraint, âme 304 dont la surface extérieure est partiellement recouverte au moyen de deux coiffes 306A, 306B formant enrobage. Ces dernières sont exécutées en un matériau rigide biocompatible tel que par exemple un acier spécial, notamment du titane, et sont assujetties à l'âme 304 par exemple par une colle de silicone.

Comme le montrent en particulier les figures 12 et 13, la section de l'âme 304 se compose de deux portions d'extrémités 308 dont la périphérie extérieure décrit un arc de cercle, qui sont reliées par deux méplats 310 formant une partie médiane 312.

La dimension transversale ou largeur D des portions d'extrémités 308 est sensiblement constante tout le long de la prothèse 302 alors que la dimension ou hauteur H des méplats 310 reliant les portions d'extrémités augmente vers l'avant de la prothèse en faisant référence à celle-ci une fois implantée dans le corps d'un patient. L'âme 304 est pourvue d'un premier et d'un second renfoncements 314, 316, dénommés encore renfoncements antérieur et postérieur. Il convient de noter que le renfoncement antérieur 314 présente des dimensions axiales supérieures et un rayon de courbure inférieur à ceux du renfoncement postérieur 316.

Ces renfoncements 314, 316 en forme de coupelle constituent des amorces de flexion, leurs dimensions respectives conférant un aspect privilégié à la flexion vers l'avant. Le renfoncement antérieur 314 est prolongé, à l'une de ses extrémités, par une extension 318 de l'âme 304, de sorte que l'une des portions d'extrémités 308 possède des dimensions longitudinales supérieures à celles lui faisant face.

La prothèse 302 est percée longitudinalement d'un orifice débouchant 318, destiné au passage d'une tige d'un outil de pose comme cela sera explicité dans ce qui suit.

Chaque coiffe 306 est réalisée sous forme d'un profilé présentant, en coupe transversale, une forme d'arc de cercle. Ces coiffes recouvrent la totalité de la périphérie extérieure des portions d'extrémités 308 de l'âme 304, alors que les méplats 310 ne sont pas recouverts. La surface extérieure de ces coiffes est munie d'un filetage 321 destiné à faciliter l'implantation de la prothèse, comme cela sera explicité dans ce qui suit.

La surface extérieure des coiffes 306 possède des irrégularités formées par exemple par gaufrage ou frittage, qui sont destinées à garantir une bonne stabilité de la prothèse une fois implantée. Les coiffes 306 sont pourvues, à leur extrémité antérieure, de rabats 322 respectifs faisant saillie l'un vers l'autre, de manière à se chevaucher partiellement à l'état libre de la prothèse. Du fait de l'extension 318, l'un 322A de ces rabats est plus éloigné de l'extrémité postérieure de la prothèse que l'autre rabat 322B. Dans un souci de clarté, ces rabats 322A, 322B seront donc dénommés respectivement distal et proximal.

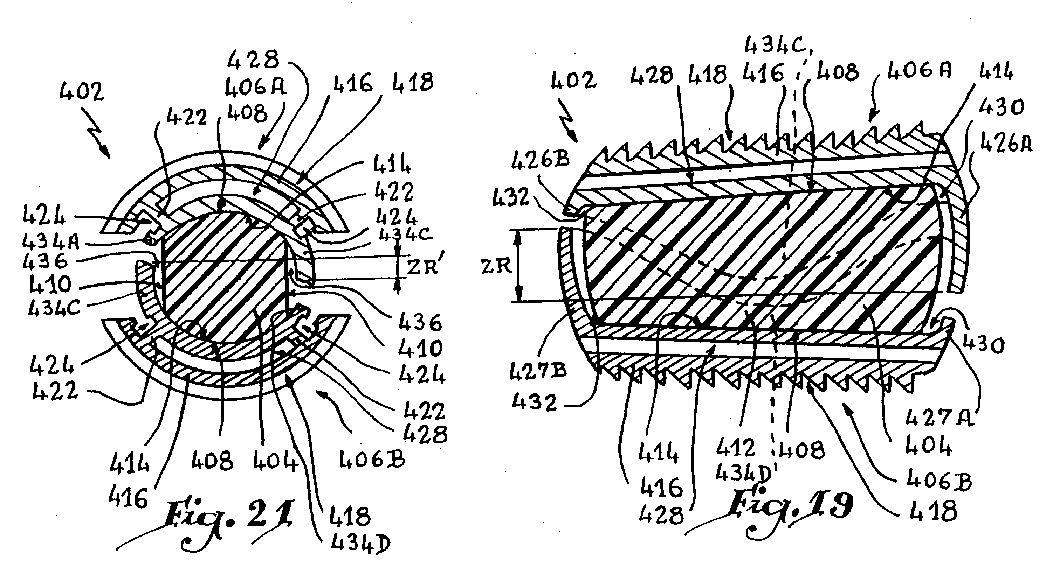

Chacun de ces rabats est pourvu d'une ouverture respective 324A, 324B de section sensiblement circulaire. L'emplacement de ces ouvertures est tel que ces dernières sont mutuellement alignées selon la direction longitudinale de la prothèse et sont co-axiales à l'orifice 320, lorsque la prothèse se trouve dans un état transversalement comprimé, comme cela sera décrit en particulier à la référence à la figure 15. La coiffe munie du rabat distal 322B est terminée par une saillie 326 s'étendant au-delà de ce rabat, à l'opposé de l'âme 304. Cette saillie 326 constitue une butée pour le rabat distal 322A, de manière à limiter le mouvement de flexion global de la partie antérieure de la prothèse.

La figure 14 représente un outil, désigné dans son ensemble par la référence 328, destiné à la pose de la prothèse représentée aux figures 11 à 14.

Cet outil 328 comprend des premier et second éléments amovibles 330, 332. Le premier élément 330 se compose d'un fût cylindrique 334 assumant une fonction de manche de préhension, terminé par deux languettes 336 à section transversale en forme d'arc de cercle, destinées à prendre appui contre les bordures des coiffes de la prothèse comme cela sera décrit dans ce qui suit. Le fût 334 est creux et annulaire et comprend un logement axial cylindrique 338.

Le second élément 332 est constitué par une tige 340 cylindrique terminée par une extrémité amincie 342 destinée à former clavette, dont la fonction sera explicitée en particulier en référence aux figures 16 et 17. La dimension transversale principale, ou largeur l, de cette extrémité 342 décroît à l'opposé de la tige 340. La tige 340 est prolongée, à l'opposé de son extrémité 342, par une portion cylindrique élargie 344, d'adaptation dans le logement 338 du fût 334. La portion d'adaptation 344 est elle-même terminée par une poignée 346. La tige 340 et la portion d'adaptation 344 sont libres de coulisser par rapport au fût 334 et de pivoter autour de l'axe principal de ce dernier.

La figure 15 illustre l'assujettissement mutuel de l'outil de pose 328 et de la prothèse 302. A cet effet, il convient tout d'abord d'insérer la tige 340 puis la portion d'adaptation 344 dans le logement annulaire 338. Puis, il s'agit de comprimer la prothèse 302, de sorte que cette dernière présente une section transversale globalement cylindrique. Une telle mise en compression peut être assurée par exemple manuellement ou au moyen d'une pince appropriée.

On introduit ensuite la tige 340, par son extrémité amincie 342, au travers de l'orifice 320 puis au sein des deux ouvertures 324A, 324B disposées dans le prolongement l'une de l'autre. L'insertion de cette tige 340 assure alors la solidarisation de la prothèse 302 et de l'outil de pose 328 dans l'état transversalement comprimé de la prothèse. En même temps que l'on a inséré la tige 340 dans les ouvertures 324A, 324B, on a déplacé longitudinalement le fût 334, de manière que les languettes 336 prennent appui contre les bordures de chaque coiffe 306A, 306B, de façon à former, avec la surface extérieure de la prothèse, une surface globalement cylindrique.

L'implantation de la prothèse dans le corps du patient s'effectue par vissage au moyen d'une action exercée sur le fût 334 formant manche. Les filetages 321 dont est pourvu la périphérie extérieure des coiffes 306 sont avantageux à l'égard de cette opération. Une fois la prothèse en place, on exerce un effort longitudinal tendant à retirer la tige 340 des ouvertures 324A, 324B, de sorte que la prothèse retrouve une configuration évasée vers sa partie antérieure, du fait de la nature de son matériau constitutif précontraint. On retire ensuite les languettes 336 par coulissement.

La figure 16 représente le positionnement mutuel des rabats 322A, 322B, une fois que la prothèse 302 a été implantée dans le corps du patient. La prothèse se trouve alors dans un état de compression intermédiaire entre son état libre représenté en référence aux figures 11 à 13 et son état comprimé en vue de la pose, représenté à la figure 15.

En effet, les vertèbres entre lesquelles elle est disposée exercent sur cette prothèse une certaine force, qui est toutefois inférieure à celle à laquelle elle est soumise durant sa pose. Il existe donc, vue en bout, une zone de recouvrement Z entre les ouvertures 324A, 324B dont sont munis les rabats 322A, 322B. La présence de cette zone de recouvrement est particulièrement avantageuse dans le cas où l'on désire retirer la prothèse, notamment en cas de descellement ou d'infection.

A cet effet, on introduit la tige 340 et la portion d'adaptation 334 au sein du fût 344, puis l'on insère l'extrémité 342 de cette tige 340 au sein de cette zone de recouvrement Z. Etant donné que la largeur l de l'extrémité 342 croît en allant vers le tige 340, les parois latérales de cette extrémité 342 viennent en butée, lors de son introduction, contre le pourtour de la zone de recouvrement, comme le montre la figure 16. On effectue alors un quart de tour au moyen de la poignée 346 solidaire de la tige 340, de manière à rapprocher l'une de l'autre les ouvertures 324A, 324B, comme cela est représenté à la figure 17. La convergence de l'extrémité 342 permet à cette dernière de s'adapter à des zones de recouvrement de dimensions différentes.

Ensuite, on exerce un effort axial de poussée sur la tige 340 afin d'engager le corps cylindrique de la tige 340 au sein des ouvertures 324A, 324B. La prothèse se trouve alors dans le même état transversalement comprimé que lors de sa pose illustrée à la figure 15.

De manière analogue, on enfile les languettes 336 le long des bordures des coiffes 306. Il est alors possible de dévisser la prothèse 302, de manière à la retirer du corps du patient. La prothèse 302 illustrée aux figures 15 à 17 est une prothèse postérieure, étant donné que son implantation est effectuée depuis le dos du patient et qu'elle doit remplacer la partie postérieure du disque.

Cependant, une telle prothèse 302 peut également être implantée depuis la face antérieure du patient afin d'être disposée à la partie antérieure, voire antéro-médiane de l'espace intervertébral. Cette implantation diffère de celle décrite ci-dessus uniquement en ce sens que, pour solidariser la tige 340 par rapport à la prothèse 302, il faut introduire cette tige tout d'abord au travers des ouvertures 324A, 324B de la prothèse, puis au sein de l'orifice 320 de cette dernière.

La prothèse 302 a été représentée avec des portions d'extrémités 308 dont la dimension transversale est sensiblement constante tout le long de cette prothèse. On peut également prévoir que ces extrémités, tout en présentant un rayon de courbure sensiblement constant tout le long de cette prothèse, s'étendent selon un secteur angulaire qui augmente continûment vers la partie antérieure de la prothèse.

Les figures 18 à 22 représentent un cinquième mode de réalisation d'une prothèse discale partielle conforme à l'invention, désignée dans son ensemble par la référence 402. Cette prothèse comprend une âme 404 réalisée en un matériau élastique bio-compatible et dont la surface extérieure est partiellement recouverte au moyen d'un enrobage formé de deux éléments 406A et 406B. Ces derniers, exécutés en un matériau rigide bio-compatible, sont assujettis à l'âme 404 par exemple au moyen d'une colle de silicone.

La section transversale de l'âme 404 se compose de deux portions d'extrémité 408 dont la périphérie extérieure décrit un arc de cercle et qui sont reliées par deux méplats 410 formant une partie médiane 412.

La dimension transversale ou largeur des portions d'extrémités 408 est sensiblement constante tout le long de la prothèse 402, alors que la hauteur des méplats 410 augmente vers l'avant de la prothèse, en faisant référence à celle-ci une fois implantée.

Chaque enrobage 406 comprend une calotte centrale 414 destinée à entrer en contact avec l'âme 404. Cette calotte 414 est reliée à une coiffe périphérique 416 réalisée sous forme d'un profilé présentant, en coupe transversale, une forme d'arc de cercle. La surface extérieure de ces coiffes est munie d'un filetage 418 destiné à faciliter l'implantation de la prothèse.

La zone de liaison entre la calotte 414 et la coiffe 416 comprend une bordure périphérique 420 s'étendant autour de la calotte et prolongée par deux pattes longitudinales 422. Ces dernières définissent, au voisinage de la bordure 420, deux glissières ou rainures longitudinales 424. Ces pattes 422 délimitent également, avec des volets d'extrémité 426, une échancrure traversante 428 en forme d'arc de cercle.

Il est prévu quatre volets, à savoir des volets avant 426A et arrière 426B pour l'enrobage 406A, et des volets avant 427A et arrière 427B pour l'enrobage 406B.

Chaque volet 426, 427 s'étend d'un des enrobages 406 vers celui qui lui est opposé, de manière sensiblement perpendiculaire à l'axe principal de la prothèse. Ces volets sont disposés de manière asymétrique.

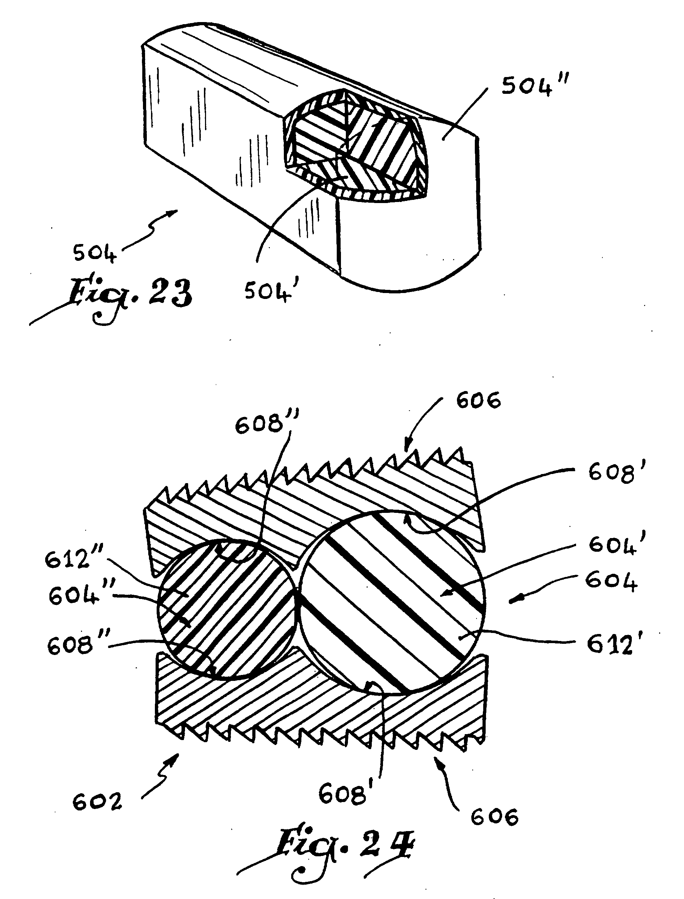

Le volet avant 426A du premier enrobage 406A et le volet opposé, à savoir le volet arrière 427B de l'autre enrobage 406B s'étendent, selon leurs dimensions principales, de façon à former une zone de recouvrement ZR. Cette dernière est particulièrement visible à la figure 21. Les projections, sur un même plan, selon l'axe principal A de la prothèse, des volets 426A et 427B possèdent une région commune, qui forme la zone de recouvrement ZR. La présence de cette dernière est de nature à réduire le cisaillement antéro-postérieur auquel est soumise la prothèse 402 une fois implantée.

Comme le montre la figure 19, chaque volet 426, 427 s'étend, dans la position de repos non comprimée de la prothèse, à distance de la paroi en regard de l'âme 404. Ceci contribue à former des volumes différentiels longitudinaux, respectivement avant 430 et arrière 432, qui limitent l'expansion de la prothèse lors de sa compression.

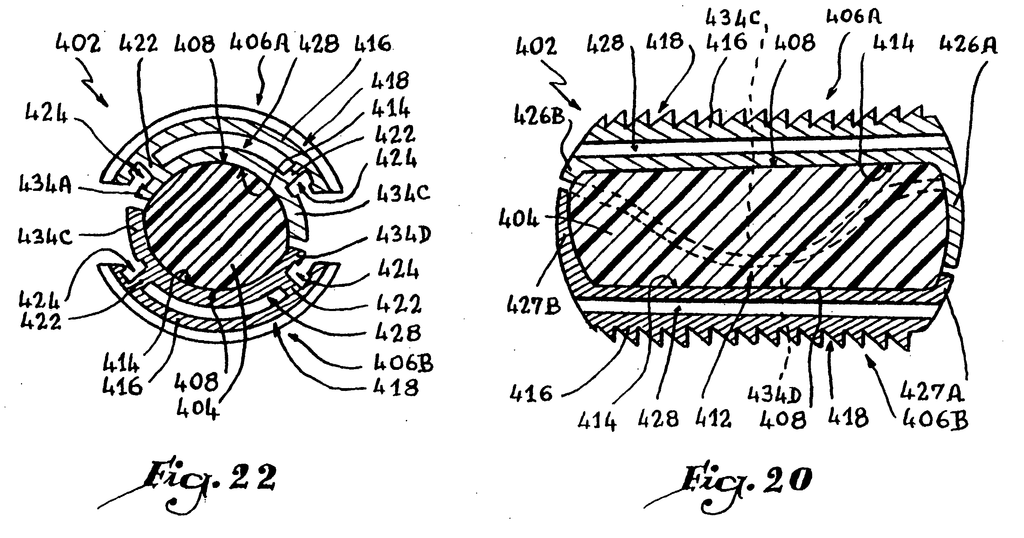

Ceci est plus particulièrement représenté à la figure 20, qui illustre la position de compression maximale de la prothèse. Dans cette position, les parois de l'âme 404, qui étaient distantes des volets 426 dans la position de repos, prennent appui contre la face intérieure de ces volets. Dans cette position comprimée, les volets en regard, à savoir d'une part 426A, 427A, et d'autre part 426B, 427B sont distants l'un de l'autre.

Chaque enrobage 406 est également pourvu de jupes latérales 434 s'étendant à partir de la bordure périphérique 420, entre chaque glissière 424 et la calotte 414. Comme le montre en particulier la figure 19, chaque jupe 434 présente, vue de côté, une hauteur variable, à savoir qu'elle s'étend selon un profil ondulé globalement sinusoïdal. Cependant, chaque jupe peut également comporter au moins un décrochement. Les jupes adjacentes, dont sont pourvus deux enrobages différents, présentent des profils sensiblement conjugués. Ainsi, les jupes 434A et 434C d'une part, ainsi que les jupes 434B et 434D d'autre part sont propres à venir en imbrication mutuelle.

En revanche, deux jupes en regard, c'est-à-dire soit 434A et 434B, soit 434C et 434D, sont disposées de façon asymétrique. Il existe donc des zones de recouvrement ZR', d'une part entre les jupes opposées 434A et 434D, d'autre part entre les jupes opposées 434B et 434C. Les projections sur un même plan, selon un axe perpendiculaire à l'axe principal de la prothèse, de chaque couple de jupes opposées, possèdent des régions communes qui forment ces zones de recouvrement. Ces dernières contribuent à réduire les effets de cisaillement latéral auquel est soumise la prothèse.

En coupe transversale, comme le montre en particulier la figure 21, chaque jupe 434 s'étend à distance des méplats 410 de l'âme 404. Ceci contribue à créer, de part et d'autre de l'âme 404, deux volumes différentiels latéraux 436. Lors de la compression maximale de la prothèse, représentée à la figure 22, l'âme 404 occupe l'ensemble de ces volumes différentiels 436, de manière à venir en contact avec la face intérieure des jupes 434. Dans cette position comprimée, il est à noter que les extrémités des jupes adjacentes, d'une part 434A et 434C, d'autre part 434B et 434D, s'étendent à distance l'une de l'autré.

La pose de la prothèse illustrée aux figures 18 à 23 s'effectue au moyen d'un outil sensiblement analogue à celui 22 décrit aux figures 4 à 8. Les glissières adjacentes, à savoir d'une part 424A et 424C, d'autre part 424B, 424D, permettent l'engagement de languettes analogues à celles 28 des figures 4 à 8. L'outil est également pourvu de languettes supplémentaires, non représentées sur ces figures 4 à 8, pénétrant dans les deux échancrures 428 dont sont pourvus les enrobages 406. Le montage de la prothèse 402 est globalement analogue à celui de la prothèse 2, illustré en référence aux figures 1 à 8.

La figure 23 illustre une âme, désignée dans son ensemble par la référence 504, susceptible de remplacer l'âme d'une prothèse décrite précédemment. Cette âme 504 est composite, à savoir qu'elle comprend un noyau 504' entouré par une enveloppe 504'', le matériau constitutif du noyau 504' étant plus compressible que celui constitutif de l'enveloppe 504''. A titre d'exemple non limitatif, le noyau est réalisé en un polymère de silicone alors que l'enveloppe 504'' est constituée de polyéthylène ou de polyuréthane.

Ce noyau 504' occupe une partie substantielle du volume de l'âme 504, et se trouve entouré par l'enveloppe sur l'ensemble de la périphérie. On peut prévoir que le noyau réalisé en un matériau compressible soit séparé de l'enveloppe externe par une succession d'habillages intermédiaires, dont les matériaux constitutifs possèdent des caractéristiques de compressibilité alternées.

On peut également prévoir de réaliser l'âme sous forme d'un noyau en un matériau plus compressible, entourée d'une enveloppe en un matériau moins compressible. Ce noyau s'étendra alors uniquement à la partie arrière de la prothèse, dont les dimensions transversales sont réduites.

L'utilisation d'une âme composite est avantageuse en ce sens qu'elle limite l'expansion de cette âme et évite le phénomène de hernies.

La figure 24 illustre un mode de réalisation supplémentaire, dans laquelle l'âme 604 comprend plusieurs éléments distincts, à savoir un élément avant 604' de plus grandes dimensions transversales, et un élément arrière 604'' de dimensions transversales restreintes. Les termes "avant" et "arrière" sont relatifs à la prothèse une fois implantée. L'élément avant 604' est réalisé en un matériau plus compressible que l'élément arrière 604". Chaque élément respectivement avant 604' et arrière 604" comprend deux portions d'extrémité transversales 608', 608'' reliées par des portions médianes respectives 612', 612".

Les deux portions d'extrémité d'un même élément sont disposées de façon globalement symétrique par rapport à un plan médian de la prothèse, qui correspond sensiblement au plan du disque intervertébral. Chaque portion d'extrémité 608', 608" est recouverte par un enrobage formant coiffe filetée 606, dont la paroi intérieure est conformée de manière à maintenir en place les éléments avant et arrière. On peut munir cette prothèse d'un enrobage analogue à celui recouvrant la prothèse des figures 18 à 22.

La prothèse conforme à l'invention permet de réaliser les objectifs précédemment mentionnés. En effet, la conformation en arc de cercle de ses portions d'extrémité, ainsi que la présence de coiffes rigides pourvues d'un filetage extérieur, assure une implantation aisée par vissage. Le fait que la prothèse présente des dimensions transversales plus importantes au niveau de sa partie avant qu'au niveau de sa partie arrière, lui confère un aspect lordosé qui se révèle avantageux d'un point de vue physiologique. La présence d'un renfoncement au niveau de la partie avant, voire de la partie arrière de la prothèse, permet à celle-ci de voir ses dimensions transversales modifiées en fonction des efforts qu'elle subit, ce qui permet une grande liberté de mouvement au patient la recevant.

Les irrégularités de la surface extérieure des coiffes garantissent une bonne stabilité de la prothèse, à la fois par frottement sur les vertèbres et du fait de la repousse osseuse qui est susceptible d'y survenir.

Le fait qu'une coiffe s'étend au-delà de l'extrémité antérieure de l'âme (figure 9) conduit à la formation d'un bras de levier qui, associé à la présence des renfoncements, assure une flexion particulièrement aisée de cette partie antérieure de la prothèse.

La présence d'une butée limitant le mouvement de la coiffe supérieure réduit en outre les risques d'expulsion postérieure de la prothèse.

La pose des prothèses représentées sur l'ensemble des figures est particulièrement aisée. En effet, étant donné que la prothèse est à même de subir une diminution sensible de ses dimensions transversales, elle est propre à être implantée aisément, sans endommager les organes au voisinage desquels elle est déplacée. De plus, la solidarisation et la désolidarisation mutuelles de la prothèse et de l'outil de pose, qui sont assurées par coulissement longitudinal, permettent un engagement aisé de la prothèse par rapport à l'outil de pose. Ce mode d'assujettissement garantit également la faculté de retirer facilement l'outil de pose de la prothèse, une fois cette dernière implantée. Etant donné que l'on retire l'outil de pose de manière longitudinale, il n'existe donc que de faibles risques d'endommager les organes au voisinage desquels on déplace cet outil.

La présence de rabats dans lesquels sont ménagées des ouvertures présentant, une fois la prothèse implantée, une zone de recouvrement, est particulièrement avantageuse. En effet, cette zone de recouvrement permet, grâce à l'insertion de l'extrémité formant clavette de la tige (figure 17), de comprimer la prothèse quand bien même l'accès direct à cette dernière est impossible pour le chirurgien. Cette mesure assure donc la possibilité de retirer la prothèse sans porter atteinte à l'intégrité physique du patient.

L'emploi de volets et/ou de jupes définissant, avec des parois en regard de l'âme, un volume différentiel d'expansion de l'âme est également avantageux. Ceci permet de conférer trois plages de compression différentes à la prothèse de l'invention. Dans une première plage, dite de charge faible, la prothèse ne se déforme sensiblement pas. Dans une deuxième plage, dite de charge moyenne, l'âme élastique se déforme de manière à occuper l'ensemble de ces volumes différentiels. Enfin, dans une troisième plage, dite de charge élevée, la prothèse est sensiblement rigide, étant donné que l'âme entre en contact, sans pouvoir être substantiellement déformée, contre les parois des jupes et/ou volets définissant ces volumes différentiels.

## Revendications

1. Prothèse discale (2 ; 102 ; 202 ; 302 ; 402 ; 602) destinée à être insérée entre deux vertèbres voisines, comprenant une âme (4 ; 104 ; 204 ; 304 ; 404 ; 504 ; 604) réalisée en un matériau élastique tel qu'un polymère de silicone ou un élastomère, recouverte, sur une partie de sa périphérie, par un enrobage (6 ; 106 ; 206 ; 306 ; 406 ; 606) réalisé en un matériau rigide et destiné à être en contact avec lesdites deux vertèbres voisines, ladite âme comprenant, en coupe transversale, deux portions d'extrémité (8 ; 108 ; 308 ; 408 ; 608', 608''), reliées par une portion médiane (12 ; 138 ; 312 ; 412 ; 612', 612"), ledit enrobage comprenant deux coiffes (6 ; 106 ; 206 ; 306 ; 416 ; 606) et recouvrant respectivement au moins partiellement la périphérie externe desdites portions d'extrémité (8 ; 108 ; 308 ; 408 ; 608', 608"), et la distance séparant lesdites coiffes augmentant vers la partie antérieure de la prothèse, **caractérisée en ce que** ladite prothèse discale est une prothèse discale partielle, et **en ce que** lesdistes coiffes sont sensiblement en arc de cercle et pourvues d'un filetage.

2. Prothèse suivant la revendication 1, **caractérisée en ce que** l'âme (4 ; 104 ; 204 ; 304 ; 404 ; 504) est réalisée de façon monobloc et possède une forme allongée.

3. Prothèse suivant l'une des revendications 1 ou 2, **caractérisée en ce que** lesdites portions d'extrémité (8 ; 108 ; 308 ; 408 ; 608', 608") sont sensiblement en arc de cercle.

4. Prothèse suivant la revendication 3, **caractérisée en ce que** la dimension transversale (D) des portions d'extrémité (8 ; 108 ; 308 ; 408) en arc de cercle est sensiblement constante tout le long de la prothèse.

5. Prothèse suivant la revendication 3, **caractérisée en ce que** lesdites portions d'extrémité présentent un rayon de courbure sensiblement constant tout le long de la prothèse et s'étendent selon un secteur angulaire qui augmente continûment vers la partie antérieure de la prothèse.

6. Prothèse suivant l'une quelconque des revendications 2 à 5, **caractérisée en ce que** la partie antérieure de l'âme (4 ; 104) est évidée de manière à former un renfoncement (20) d'amorce de flexion.

7. Prothèse suivant l'une quelconque des revendications 2 à 6, **caractérisée en ce que** la partie postérieure de la prothèse est pourvue d'un second renfoncement d'amorce de flexion, dont les dimensions axiales sont sensiblement inférieures à celles du premier renfoncement (20).

8. Prothèse suivant l'une quelconque des revendications 2 à 7, **caractérisée en ce que** la partie médiane (12 ; 312 ; 412) comprend deux méplats (10 ; 310 ; 410) reliant les extrémités voisines des deux portions d'extrémité (8 ; 308 ; 408).

9. Prothèse suivant l'une quelconque des revendications 2 à 7, **caractérisée en ce que** la partie médiane (138) comprend deux gorges (136) reliant les extrémités voisines des deux portions d'extrémité (108).

10. Prothèse suivant l'une quelconque des revendications 2 ou 9, l'âme monobloc (504) comprend un noyau (504') réalisé en un premier matériau et une enveloppe (504") réalisée en un second matériau moins compressible que le premier matériau.

11. Prothèse suivant la revendication 1, **caractérisée en ce que** l'âme (604) comprend plusieurs éléments (604', 604").

12. Prothèse suivant la revendication 11, **caractérisée en ce que** l'âme (604) comprend un élément arrière (604") et un élément avant (604') plus compressible que l'élément arrière et dont les dimensions transversales sont supérieures à celle de l'élément arrière.

13. Prothèse suivant l'une quelconque des revendications précédentes, **caractérisée en ce qu'**au moins l'une des coiffes (106A ; 206A ; 406A, 406B) s'étend au-delà de l'extrémité antérieure de la portion d'extrémité (108) qu'elle recouvre.

14. Prothèse suivant l'une quelconque des revendications précédentes, **caractérisée en ce que** les coiffes (6 ; 106 ; 416) sont pourvues, au voisinage de chacune de leurs bordures (18), de rainures longitudinales (16 ; 424) propres à être enfilées par coulissement dans des nervures (30) correspondantes dont est muni un outil de pose.

15. Prothèse suivant l'une quelconque des revendications précédentes, **caractérisée en ce qu'**elle est pourvue d'une butée (240) faisant saillie, depuis le voisinage de l'extrémité avant d'une première coiffe (206B), en direction d'une seconde coiffe (206A), ladite butée étant apte à limiter le mouvement de ladite seconde coiffe (206A) en direction de ladite première coiffe (206B);

16. Prothèse suivant la revendication 15, **caractérisée en ce que** ladite butée (240) limite le mouvement de ladite seconde coiffe (206A) selon un angle prédéterminé (α) par rapport à un axe (A') parallèle à l'axe (A) de ladite première coiffe (206B).

17. Prothèse suivant l'une quelconque des revendications précédentes, **caractérisée en ce que** ladite prothèse est pourvue de moyens (426, 427) de réduction du cisaillement antéro-postérieur.

18. Prothèse suivant la revendication 17, **caractérisée en ce que** les moyens de réduction du cisaillement antéro-postérieur comprennent au moins un volet avant (426A) et un volet arrière (427B), dont est pourvu l'enrobage (406), lesdits volets s'étendant au voisinage des extrémités respectivement avant et arrière de ladite âme (404) et possédant une zone de recouvrement (ZR).

19. prothèse suivant la revendication 18, **caractérisée en ce qu'**au moins un desdits volets (426, 427) s'étend, au moins en partie, à distance d'une paroi d'extrémité correspondante de ladite âme, de manière à former au moins un volume différentiel (430, 432) longitudinal avec ladite âme (404).

20. Prothèse suivant l'une quelconque des revendications précédentes, **caractérisée en ce que** ladite prothèse est pourvue de moyens (434) de réduction du cisaillement latéral.

21. Prothèse suivant la revendication 20, **caractérisée en ce que** les moyens de réduction du cisaillement latéral comprennent au moins des première (434A, 434C) et seconde (434B, 434D) jupes latérales opposées, dont est pourvu l'enrobage (406), lesdites jupes (434) s'étendant au voisinage des parois latérales de ladite âme et possédant une zone de recouvrement (ZR') .

22. Prothèse suivant la revendication 21, **caractérisée en ce que** lesdites jupes présentent, en vue de côté, un bord non rectiligne, en particulier un bord formant au moins une ondulation ou au moins un décrochement.

23. Prothèse suivant l'une des revendications 21 ou 22, **caractérisée en ce qu'**au moins une desdites jupes (434) s'étend, au moins en partie, à distance d'une paroi latérale correspondante de ladite âme (404), de manière à former au moins un volume différentiel latéral (436) avec ladite âme.

24. Prothèse suivant l'une quelconque des revendications précédentes, **caractérisée en ce que** ledit enrobage (406) comprend une calotte (414) de contact avec l'âme (404), séparée desdites coiffes par une zone de liaison (420, 422) échancrée.

25. Prothèse suivant l'une quelconque des revendications précédentes, **caractérisée en ce que** la prothèse est pourvue d'un orifice longitudinal (320) de passage d'une tige (340) d'un outil de pose (328).

26. Prothèse suivant la revendication 25, **caractérisée en ce qu'**elle est pourvue de moyens de recompression (322A, 322B, 324A, 324B) , propres à la ramener dans un état transversalement comprimé après sa pose.

27. Prothèse suivant la revendication 26, **caractérisée en ce que** les moyens de recompression comprennent deux rabats (322A, 322B) décalés longitudinalement et faisant saillie l'un vers l'autre à partir du voisinage de la partie antérieure de chaque coiffe (306A, 306B), lesdits rabats étant pourvus d'ouvertures (324A, 324B) respectives de passage de la tige (340) de l'outil de pose (328), alignées longitudinalement dans l'état transversalement comprimé de la prothèse.

28. Prothèse suivant la revendication 27, **caractérisée en ce que** lesdites ouvertures (324A, 324B) des rabats (322A, 322B) sont sensiblement circulaires et il existe, lorsque la prothèse est insérée entre les deux vertèbres voisines, une zone de recouvrement (Z) entre ces deux ouvertures, ladite zone de recouvrement présentant un profil transversal globalement ovale et étant propre à recevoir une extrémité aplatie (342) de ladite tige (340) dudit outil de pose (328).

29. Outil de pose pour la prothèse suivant l'une quelconque des revendications précédentes, **caractérisé en ce qu'**il comprend un manche de préhension (24 ; 334) prolongé par des moyens (28, 30 ; 336 ; 340) propres à solidariser ladite prothèse par rapport audit outil dans un état transversalement comprimé de ladite prothèse, et **en ce que** ledit manche (24) est prolongé car deux languettes (28, 336) à section en forme d'arc de cercle, dans le volume intérieur desquelles la prothèse (2, 302) est destinée à être engagée, la surface extérieure des languettes (2, 336) formant, avec la surface extérieure de la prothèse (2, 302) une fois engagée, une surface globalement cylindrique.

30. Outil de pose suivant la revendication 29, **caractérisé en ce que** lesdits moyens de solidarisation comprennent des moyens de fixation (30) par enfilement.

31. Outil de pose suivant la revendication 30, **caractérisé en ce que** lesdits moyens de fixation par enfilement comprennent des nervures (30) dont est pourvu l'outil de pose (22) ou la prothèse (2), coopérant avec des rainures (16) ménagées dans la prothèse ou l'outil de pose.

32. Outil de pose suivant la revendication 29 pour la pose d'une prothèse selon l'une quelconque des revendications 25 à 28, **caractérisé en ce que** lesdits moyens de solidarisation comprennent une tige (340) propre à pénétrer dans l'orifice longitudinal (320) dont est pourvue la prothèse (302).

33. Outil de pose suivant la revendication 32, **caractérisé en ce que** la tige (340) est propre à pénétrer dans les ouvertures (324A, 324B) dont sont munis les rabats (322A, 322B).

34. Outil de pose suivant l'une des revendications 32 ou 33, **caractérisé en ce que** ladite tige (340) est libre de pivoter par rapport audit manche (334).

35. Outil de pose suivant la revendication 34, **caractérisé en ce que** la tige (340) présente un profil transversal sensiblement circulaire et possède une extrémité distale aplatie (342) qui est propre à pénétrer dans ladite zone de recouvrement (Z) entre les ouvertures (324A, 324B) desdits rabats (322A, 322B).

## Patentansprüche

1. Bandscheibenprothese (2; 102; 202; 302; 402; 602), die dazu bestimmt ist, zwischen zwei benachbarte Wirbel eingesetzt zu werden, die ein Innenteil (4; 104; 204; 304; 404; 504; 604) umfasst, das in einem elastischen Material, wie etwa einem Silikonpolymer oder einem Elastomer, ausgeführt ist, das auf einem Teil seines Umfanges mit einer Ummantelung (6; 106; 206; 306; 406; 606) bedeckt ist, die in einen steifen Material ausgeführt ist, und die dazu bestimmt ist, mit den zwei benachbarten Wirbeln in Kontakt zu stehen, wobei das Innenteil im Querschnitt zwei äußere Abschnitte (8; 108; 308; 408; 608'; 608") umfasst, die durch einen mittleren Abschnitt (12; 138; 312; 412; 612', 612'') verbunden sind, wobei die Ummantelung zwei Kappen (6; 106; 206; 306; 416; 606) umfasst, und jeweils wenigstens teilweise die Außenfläche der äußeren Abschnitte (8; 108; 308; 408; 608', 608'') bedeckt, und wobei der Abstand zwischen den Kappen in Richtung des vorderen Teils der Prothese ansteigt, **dadurch gekennzeichnet, dass** die Bandscheibenprothese eine partielle Bandscheibenprothese ist, und dadurch, dass die Kappen im wesentlichen kreisbogenförmig sind und mit einem Gewinde versehen sind.

2. Prothese nach Anspruch 1, **dadurch gekennzeichnet, dass** das Innenteil (4; 104; 204; 304; 404; 504) in einem Stück ausgeführt ist und eine längliche Form hat.

3. Prothese nach einem der Ansprüche 1 oder 2, **dadurch gekennzeichnet, dass** die äußeren Abschnitte (8; 108; 308; 408; 608', 608") im wesentlichen kreisbogenförmig sind.

4. Prothese nach Anspruch 3, **dadurch gekennzeichnet, dass** die Querabmessung (D) der kreisbogenförmigen äußeren Abschnitte (8; 108; 308; 408) über die ganze Länge der Prothese im wesentlichen konstant ist.

5. Prothese nach Anspruch 3,**dadurch gekennzeichnet, dass** die äußeren Abschnitte über die ganze Länge der Prothese einen im wesentlichen konstanten Krümmungsradius aufweisen und sich über einen Winkelbereich erstrecken, der sich in Richtung des vorderen Teils der Prothese kontinuierlich vergrößert.

6. Prothese nach irgendeinem der Ansprüche 2 bis 5, **dadurch gekennzeichnet, dass** der vordere Teil des Innenteils (4; 104) derart ausgespart ist, dass er eine Vertiefung (20) für den Anfangsbereich bei Biegebeanspruchung bildet.

7. Prothese nach irgendeinem der Ansprüche 2 bis 6, **dadurch gekennzeichnet, dass** der hintere Teil der Prothese mit einer zweiten Vertiefung für den Anfangsbereich bei Biegebeanspruchung versehen ist, deren axiale Abmessungen im wesentlichen kleiner als die der ersten Vertiefung (20) sind.

8. Prothese nach irgendeinem der Ansprüche 2 bis 7, **dadurch gekennzeichnet, dass** der mittlere Abschnitt (12; 312; 412) zwei Abflachungen (10; 310;. 410) umfasst, die die Enden verbinden, die an die zwei äußeren Abschnitte (8; 308; 408) angrenzen.

9. Prothese nach irgendeinem der Ansprüche 2 bis 7, **dadurch gekennzeichnet, dass** der mittlere Abschnitt (138) zwei Auskerbungen (136) umfasst, die die Enden verbinden, die an die zwei äußeren Abschnitte (108) angrenzen.

10. Prothese nach irgendeinem der Ansprüche 2 bis 9, bei der das in einem Stück ausgeführte Innenteil (504) einen Kern (504'), der in einem ersten Material ausgeführt ist, und einen Mantel (504'') umfasst, der in einem zweiten Material ausgeführt ist, das weniger kompressibel als das erste Material ist.

11. Prothese nach Anspruch 1, **dadurch gekennzeichnet, dass** das Innenteil (604) mehrere Elemente (604', 604") umfasst.

12. Prothese nach Anspruch 11, **dadurch gekennzeichnet, dass** das Innenteil (604) ein hinteres Element (604'') und ein vorderes Element (604') umfasst, das kompressibler als das hintere Element ist, und dessen Querabmessungen größer als die des hinteren Elements sind.

13. Prothese nach irgendeinem der vorangehenden Ansprüche, **dadurch gekennzeichnet, dass** sich wenigstens eine der Kappen (106A, 206A, 406A; 406B) von dem hinteren Ende des Abschnitts am Ende (108) aus erstreckt, den sie bedeckt.

14. Prothese nach irgendeinem der vorangehenden Ansprüche, **dadurch gekennzeichnet, dass** die Kappen (6; 106; 416) in der Nähe von jedem ihrer Ränder (18) mit Längsnuten (16; 424) versehen sind, die dazu geeignet sind, durch Aufschieben auf entsprechende Rippen (30) aufgesteckt zu werden, mit denen ein Einsetzwerkzeug ausgerüstet ist.

15. Prothese nach irgendeinem der vorangehenden Ansprüche, **dadurch gekennzeichnet, dass** sie mit einem Anschlag (240) versehen ist, der in der Nähe des vorderen Endes einer ersten Kappe (206B) in Richtung einer zweiten Kappe (206A) vorspringt, wobei der Anschlag dazu geeignet ist, die Bewegung der zweiten Kappe (206A) in Richtung der ersten Kappe (206B) zu begrenzen.

16. Prothese nach Anspruch 15, **dadurch gekennzeichnet, dass** der Anschlag (240) die Bewegung der Kappe (206A) auf einen vorher festgelegten Winkel (α) bezüglich einer Achse (A') begrenzt, die parallel zur Achse (A) der ersten Kappe (206B) ist.

17. Prothese nach irgendeinem der vorangehenden Ansprüche, **dadurch gekennzeichnet, dass** die Prothese mit Mitteln (426, 427) zur Verringerung der Schubbeanspruchung in vorwärts-rückwärts-Richtung versehen ist.

18. Prothese nach Anspruch 17, **dadurch gekennzeichnet, dass** die Mittel zur Verringerung der Schubbeanspruchung in vorwärts-rückwärts-Richtung wenigstens einen vorderen Flügel (426A) und einen hinteren Flügel (427B) umfassen, mit dem die Ummantelung versehen ist, wobei sich die Flügel jeweils in der Nähe des vorderen und hinteren Endes des Innenteils (404) erstrecken und einen Überdeckungsbereich (ZR) besitzen.

19. Prothese nach Anspruch 18, **dadurch gekennzeichnet, dass** sich wenigstens einer der Flügel (426, 427) wenigstens teilweise in einem Abstand von einer Stirnwand, die zu dem Innenteil gehört, derart erstreckt, dass er wenigstens einen Zwischenraum (430, 432) in Längsrichtung mit dem Innenteil (404) bildet.

20. Prothese nach irgendeinem der vorangehenden Ansprüche, **dadurch gekennzeichnet, dass** die Prothese mit Mitteln (434) zur Verringerung der seitlichen Schubbeanspruchung versehen ist.

21. Prothese nach Anspruch 20, **dadurch gekennzeichnet, dass** die Mittel zur Reduktion der seitlichen Schubbeanspruchung wenigstens erste (434A, 434C) und zweite (434B, 434D) gegenüberliegende seitliche Schürzen umfassen, mit denen die Ummantelung (406) versehen ist, wobei sich die Schürzen (434) in der Nähe der Seitenwand des Innenteils erstrecken und einen Überdeckungsbereich (ZR') besitzen.

22. Prothese nach Anspruch 21, **dadurch gekennzeichnet, dass** die Schürzen in der Seitenansicht einen nichtgeradlinigen Rand aufweisen, insbesondere einen Rand, der wenigstens eine Welle oder wenigstens einen Absatz bildet.

23. Prothese nach einem der Ansprüche 21 oder 22, **dadurch gekennzeichnet, dass** sich wenigstens eine der Schürzen (434) wenigstens teilweise in einem Abstand zur entsprechenden Seitenwand des Innenteils (404) derart erstreckt, dass sie wenigstens einen Zwischenraum in seitlicher Richtung (436) mit dem Innenteil bildet.

24. Prothese nach irgendeinem der vorangehenden Ansprüche, **dadurch gekennzeichnet, dass** die Ummantelung (406) eine Halbschale (414) zur Verbindung mit dem Innenteil (404) umfasst, die von den Kappen durch einen bogenförmig ausgeschnittenen Verbindungsbereich (420, 422) getrennt ist.

25. Prothese nach irgendeinem der vorangehenden Ansprüche, **dadurch gekennzeichnet, dass** die Prothese mit einer Öffnung in Längsrichtung (320) versehen ist, um einen Stift (340) eines Einsetzwerkzeugs (328) durchzulassen.

26. Prothese nach Anspruch 25, **dadurch gekennzeichnet, dass** sie mit Mitteln zum erneuten Zusammenpressen (322A, 322B, 324A, 324B) versehen ist, die dazu geeignet sind, sie nach ihrem Einsetzen in einen quer komprimierten Zustand zurückzuversetzen.

27. Prothese nach Anspruch 26, **dadurch gekennzeichnet, dass** die Mittel zum erneuten Zusammenpressen zwei Laschen (322A, 322B) umfassen, die in Längsrichtung versetzt sind und wobei eine in Richtung der anderen in der Nähe des vorderen Teils der Kappe (306A, 306B) vorspringt, wobei die Laschen jeweils mit Öffnungen (324A, 324B) für den Durchgang des Stiftes (340) des Einsetzwerkzeugs (328) versehen sind, die im komprimierten Zustand der Prothese in Längsrichtung fluchten.

28. Prothese nach Anspruch 27, **dadurch gekennzeichnet, dass** die Öffnungen (324A, 324B) der Laschen (322A, 322B) im wesentlichen kreisförmig sind und, wenn die Prothese zwischen zwei benachbarten Wirbeln eingesetzt ist, ein Überdeckungsbereich (Z) der beiden öffnungen existiert, wobei der überdeckungsbereich im Ganzen genommen in Querrichtung ein ovales Profil aufweist, das dazu geeignet ist, ein abgeflachtes Ende (342) des Stiftes (340) des Einsetzwerkzeugs (328) aufzunehmen.

29. Einsetzwerkzeug für die Prothese nach irgendeinem der vorangehenden Ansprüche, **dadurch gekennzeichnet, dass** es einen Handgriff (24; 334) umfasst, der durch Mittel (28, 30; 336; 340) verlängert ist, die dazu geeignet sind, die Prothese in Bezug auf das Werkzeug in einem quer komprimierten Zustand der Prothese zusammenzuhalten und dadurch, dass der Griff (24) durch zwei Zungen (28, 336) mit einem Querschnitt in Form eines Kreisbogens verlängert ist, wobei deren Innenraum dazu bestimmt ist, die Prothese (302) einzuschieben, wobei die Außenfläche der Zungen (28, 336) mit der Außenfläche der Prothese (2, 302), wenn sie einmal eingesetzt ist, eine im Ganzen gesehen zylindrische Oberfläche bildet.

30. Einsetzwerkzeug nach Anspruch 29, **dadurch gekennzeichnet, dass** die Mittel zum Zusammenhalten Mittel (30) zum Verbinden durch Einstecken umfassen.

31. Einsetzwerkzeug nach Anspruch 30, **dadurch gekennzeichnet, dass** die Mittel zum Verbinden durch Einstecken Rippen (30) umfassen, mit denen das Einsetzwerkzeug (22) oder die Prothese (2) versehen ist, die mit den Nuten (16) zusammenarbeiten, die in der Prothese oder in dem Einsetzwerkzeug angeordnet sind.

32. Einsetzwerkzeug nach Anspruch 29 für das Einsetzen einer Prothese nach irgendeinem der Ansprüche 25 bis 28, **dadurch gekennzeichnet, dass** die Mittel zum Zusammenhalten einen Stift (340) umfassen, der dazu geeignet ist, durch die Öffnung in Längsrichtung (320) gesteckt zu werden, mit der die Prothese (302) versehen ist.

33. Einsetzwerkzeug nach Anspruch 32, **dadurch gekennzeichnet, dass** der Stift (340) dazu geeignet ist, durch die Öffnungen (324A, 324B) gesteckt zu werden, mit denen die Laschen (322A, 322B) versehen sind.

34. Einsetzwerkzeug nach einem der Ansprüche 32 oder 33, **dadurch gekennzeichnet, dass** der Stift (340) bezüglich des Griffs (334) frei drehbar ist.

35. Einsetzwerkzeug nach Anspruch 34, **dadurch gekennzeichnet, dass** der Stift (340) in Querrichtung ein im Wesentlichen kreisrundes Profil aufweist, und ein abgeflachtes vorderes Ende(342) hat, das dazu geeignet ist, durch den Überdeckungsbereich (Z) der Öffnungen (324A, 324B) der Laschen (322A, 322B) gesteckt zu werden .

## Claims

1. Disc prosthesis (2; 102; 202; 302; 402; 602) which is to be inserted between two adjacent vertebrae, comprising a core (4; 104; 204; 304; 404; 504; 604) which is produced from a resilient material, such as a silicone polymer or an elastomer, and which is covered, over a portion of its periphery, by a casing (6; 106; 206; 306; 406; 606) which is produced from a rigid material and which is to be in contact with the said two adjacent vertebrae, the core comprising, in cross-section, two end portions (8; 108; 308; 408; 608', 608") connected by a median portion (12; 138; 312; 412; 612', 612"), the casing comprising two caps (6; 106; 206; 306; 416; 606) and covering, respectively, at least partially the outer periphery of the end portions (8; 108; 308; 408; 608', 608"), and the distance separating the caps increasing towards the anterior portion of the prosthesis, **characterised in that** the disc prosthesis is a partial disc prosthesis and **in that** the caps are substantially in the shape of an arc of a circle and are provided with a thread.

2. Prosthesis according to claim 1, **characterised in that** the core (4; 104; 204; 304; 404; 504) is produced in a monobloc manner and has an elongate shape.

3. Prosthesis according to either claim 1 or claim 2, **characterised in that** the end portions (8; 108; 308; 408; 608', 608") are substantially in the shape of an arc of a circle.

4. Prosthesis according to claim 3, **characterised in that** the transverse dimension (D) of the end portions (8; 108; 308; 408) in the shape of an arc of a circle is substantially constant over the entire length of the prosthesis.

5. Prosthesis according to claim 3, **characterised in that** the end portions have a radius of curvature which is substantially constant over the entire length of the prosthesis and they extend in accordance with an angular sector which increases continuously towards the anterior portion of the prosthesis.

6. Prosthesis according to any one of claims 2 to 5, **characterised in that** the anterior portion of the core (4; 104) is hollowed out in such a manner as to form a recess (20) for initiating bending.

7. Prosthesis according to any one of claims 2 to 6, **characterised in that** the posterior portion of the prosthesis is provided with a second recess for initiating bending, the axial dimensions of which are substantially smaller than those of the first recess (20).

8. Prosthesis according to any one of claims 2 to 7, **characterised in that** the median portion (12; 312; 412) comprises two flat surfaces (10; 310; 410) connecting the adjacent ends of the two end portions (8; 308; 408).

9. Prosthesis according to any one of claims 2 to 7, **characterised in that** the median portion (138) comprises two grooves (136) connecting the adjacent ends of the two end portions (108).

10. Prosthesis according to either claim 2 or claim 9, the monobloc core (504) comprises an inner core (504') produced from a first material and a covering (504") produced from a second material which is less compressible than the first material.

11. Prosthesis according to claim 1, **characterised in that** the core (604) comprises several members (604', 604").

12. Prosthesis according to claim 11, **characterised in that** the core (604) comprises a rear member (604") and a front member (604') which is more compressible than the rear member and the transverse dimensions of which are larger than those of the rear member.

13. Prosthesis according to any one of the preceding claims, **characterised in that** at least one of the caps (106A; 206A; 406A, 406B) extends beyond the anterior end of the end portion (108) which it covers.

14. Prosthesis according to any one of the preceding claims, **characterised in that** the caps (6; 106; 416) are provided, in the vicinity of each of their borders (18), with longitudinal channels (16; 424) which are capable of being engaged by sliding in corresponding ribs (30) with which a positioning tool is provided.

15. Prosthesis according to any one of the preceding claims, **characterised in that** it is provided with a stop (240) which projects from the vicinity of the front end of a first cap (206B) in the direction towards a second cap (206A), the stop being capable of limiting the movement of the second cap (206A) in the direction towards the first cap (206B).

16. Prosthesis according to claim 15, **characterised in that** the stop (240) limits the movement of the second cap (206A) in accordance with a predetermined angle (α) relative to an axis (A') parallel with the axis (A) of the first cap (206B).

17. Prosthesis according to any one of the preceding claims, **characterised in that** the prosthesis is provided. with means (426, 427) for reducing antero-posterior shearing.

18. Prosthesis according to claim 17, **characterised in that** the means for reducing antero-posterior shearing comprise at least one front flap (426A) and one rear flap (427B), with which the casing (406) is provided, the flaps extending in the vicinity of the front and rear ends, respectively, of the core (404) and having an overlapping region (ZR).

19. Prosthesis according to claim 18, **characterised in that** at least one of the flaps (426, 427) extends, at least partially, at a distance from a corresponding end wall of the core in order to form at least one longitudinal differential volume (430, 432) with the core (404).

20. Prosthesis according to any one of the preceding claims, **characterised in that** the prosthesis is provided with means (434) for reducing lateral shearing.

21. Prosthesis according to claim 20, **characterised, in that** the means for reducing lateral shearing comprise at least first (434A, 434C) and second (434B, 434D) opposite lateral skirts, with which the casing (406) is provided, the skirts (434) extending in the vicinity of the lateral walls of the core and having an overlapping region (ZR').

22. Prosthesis according to claim 21, **characterised in that** the skirts have, in side view, a non-rectilinear edge, in particular an edge forming at least one corrugation or at least one step.

23. Prosthesis according to either claim 21 or claim 22, **characterised in that** at least one of the skirts (434) extends, at least partially, at a distance from a correspending lateral wall of the core (404) in order to form at least one lateral differential volume (436) with the core.

24. Prosthesis according to any one of the preceding claims, **characterised in that** the casing (406) comprises a hood (414) for contact with the core (404), which hood is separated from the caps by a scalloped connecting region (420, 422).

25. Prosthesis according to any one of the preceding claims, **characterised in that** the prosthesis is provided with a longitudinal orifice (320) for the passage of a rod (340) of a positioning tool (328).

26. Prosthesis according to claim 25, **characterised in that** it is provided with recompression means (322A, 322B, 324A, 324B) capable of returning it to a transversely compressed state after it has been put in position.

27. Prosthesis according to claim 26, **characterised in that** the recompression means comprise two flaps (322A, 322B) which are offset longitudinally and project towards one another starting from the vicinity of the anterior portion of each cap (306A, 306B), the flaps being provided with respective openings (324A, 324B) for the passage of the rod (340) of the positioning tool (328), which openings are aligned longitudinally in the transversely compressed state of the prosthesis.

28. Prosthesis according to claim 27, **characterised in that** the openings (324A, 324B) of the flaps (322A, 322B) are substantially circular and, when the prosthesis is inserted between the two adjacent vertebrae, there is an overlapping region (Z) between those two openings, the overlapping region having an overall oval transverse profile and being capable of receiving a flattened end (342) of the rod (340) of the positioning tool (328).

29. Positioning tool for the prosthesis according to any one of the preceding claims, **characterised in that** it comprises a gripping handle (24; 334) extended by means (28, 30; 336; 340) capable of securing the prosthesis relative to the tool in a transversely compressed state of the prosthesis, and **in that** the handle (24) is extended by two tongues (28, 336) which have a cross-section in the shape of an arc of a circle and in the internal volume of which the prosthesis (2, 302) is to be engaged, the outer surface of the tongues (28, 336) forming, with the outer surface of the prosthesis (2, 302) once engaged, an overall cylindrical surface.

30. Positioning tool according to claim 29, **characterised in that** the securing means comprise means (30) for fixing by engagement.

31. Positioning tool according to claim 30, **characterised in that** the means for fixing by engagement comprise ribs (30) with which the positioning tool (22) or the prosthesis (2) is provided, and which cooperate with channels (16) formed in the prosthesis or the positioning tool.

32. Positioning tool according to claim 29 for positioning a prosthesis according to any one of claims 23 to 28, **characterised in that** the securing means comprise a rod (340) capable of penetrating into the longitudinal orifice (320) with which the prosthesis (302) is provided.

33. Positioning tool according to claim 32, **characterised in that** the rod (340) is capable of penetrating into the openings (324A, 324B) with which the flaps (322A, 322B) are provided.

34. Positioning tool according to either claim 32 or claim 33, **characterised in that** the rod (340) is free to pivot relative to the handle (334).

35. Positioning tool according to claim 34, **characterised in that** the rod (340) has a substantially circular transverse profile and has a flattened distal end (342) which is capable of penetrating into the overlapping region (Z) between the openings (324A, 324B) of the flaps (322A, 322B).
